# EUROPEAN PATENT APPLICATION

(11) **EP 4 574 989 A1**
(43) Date of publication of application: **25.06.2025**
(21) Application number: 23220190.5
(22) Date of filing: 22.12.2023
(51) Int. Cl.: C12P 19/18, C12P 19/38, C12P 19/40

(54) **ALPHA-L-FUCOSIDASES AND USES THEREOF**

(71) Applicant: Vilnius University, 01513 Vilnius (LT)
(72) Inventor: Stankeviciute, Jonita, Vilnius (LT); Meskys, Rolandas, Vilnius (LT); Krupinskaite, Agne, Vilnius (LT); Stanislauskiene, Ruta, Vilnius (LT); Rutkiene, Rasa, Vilnius (LT); Urbeliene, Nina, Vilnius (LT)
(74) Representative: AAA Law

(57) **Abstract**

The present invention relates to a method for preparing fucosylated nucleosides comprising the step of enzymatic transfucosylation and the fucosylated nucleosides obtainable by the method. The method is disclosed for utilizing alpha-L-fucosidases *in vitro* for the preparation of fucosylated ribonucleosides.

## Description

### FIELD OF THE INVENTION

This invention relates to the technical field of biochemistry, more specifically to enzymes called alpha-L-fucosidases and their use in the synthesis of fucosylated nucleosides.

### BACKGROUND OF THE INVENTION

Nucleosides are composed of nucleobase and a covalently attached sugar composed of five carbon atoms. A nitrogenous base can either be pyrimidine or purine. Pyrimidine is a six-membered aromatic heterocyclic nitrogenous base with two nitrogen atoms at positions 1 and 3. Purine is an aromatic heterocyclic nitrogenous base comprised of pyrimidine and imidazole rings. The fifth carbon atom of sugar is attached to the nitrogenous base at the primary carbon atom through an *N*-β-glycosidic bond. If a nucleoside contains purine nucleobase, a bond with sugar is formed at the N-9 atom. If a nucleoside contains pyrimidine nitrogenous base bond with sugar is formed at the N-1 atom. Sugar can either be D-ribose or 2'-deoxy-D-ribose. If sugar is D-ribose, the hydroxyl group is present at the second position. If sugar is 2'-deoxy-D-ribose, the hydroxyl group is not present at the second position. Both D-ribose and 2'-deoxy-D-ribose have a close ring structure and β-furanose form.

As components of nucleotides, nucleosides are considered to be the structural unit of DNA and RNA nucleic acids. Additionally, nucleosides take part in various other metabolic processes, and novel synthetic nucleosides and their analogues have therapeutic, such as antiviral or anticancer, properties (Guinan et al., 2020; Yates and Seley-Radtke, 2019). The modifications to the sugar moiety, the presence or absence of specific functional groups, and the nature of the nucleobase all contribute to the selectivity and efficacy of therapeutic compounds. For example, azidothymidine is used in HIV prevention as a reverse transcriptase inhibitor (Liu et al., 2020), while trifluorthymidine has antiviral properties suitable for herpes treatment (Sibley and Larkin, 2020). Anticancer properties can be found in 2'-deoxy-2',2'-difluorocytidine, also known as gemcitabine, which is used as a chemotherapy medication (loka et al., 2022). In the case of uridine analogues, idoxuridine and trifluorothymidine, both compounds have antiviral properties. Nevertheless, the specific functional group at the 5^{th} position determines the properties of the compound as idoxuridine has iodine at position 5 and it can be administered only topically due to cardiotoxicity, whereas trifluorothymidine has a trifluoromethyl group at position 5 and is not cardiotoxic.

The importance of the sugar moiety could be exemplified by an adenosine analogue 9-β-D-arabinofuranosyladenine, known as vidarabine, which has D-ribose replaced with D-arabinose which leads to a different hydroxyl group configuration at the C-2 position. This stereoisomeric change leads to antiviral therapeutic properties (Siedentop and Rosenthal, 2022). Therefore, stereospecificity could be considered an especially important parameter in therapeutic compound synthesis. This only proves that there is a need for tools capable of synthesizing these specific nucleoside analogues with a certain stereospecificity.

Alpha-L-fucosidases are enzymes that catalyze the removal of fucose residues from fucosylated compounds. They are also capable of catalysing transfucosylation. Transfucosylation refers to the enzymatic transfer of fucose residues from a donor substrate to an acceptor substrate, resulting in the production of novel fucosylated compounds.

To date, it has not been estimated that alpha-L-fucosidases may catalyse the transfucosylation of nucleosides forming fucosylated nucleoside analogues.

Chemical synthesis of glycosylated nucleoside analogues is burdensome (consists of many reaction steps) and requires many protective steps to achieve the stereospecificity of the modifications. The synthesis of disaccharide nucleosides utilizing the protection of the 2',3'-cis-diol of ribonucleosides by a boronic ester has been previously described. The glycosylation of the uridine, which was protected by a boronic acid, with the thioglycoside followed by acetylation gave the disaccharide nucleoside with a 1 ",5'-glycosidic linkage. This chemical synthesis method was used for the glycosylation of protected or unprotected adenosine, guanosine, uridine or cytidine, with the galactosyl donor. In the case of the unprotected nucleosides, the complex mixtures of the glycosylated products were observed. *O*-Glycosylations of 5-fluorouridine with the glucosyl donor, the galactosyl donor, and the mannosyl donor were also conducted (Someya et al., 2017). However, this synthesis method of disaccharide nucleosides is a multi-step, the anomeric mixtures are produced and, to perform regioselective *O*-glycosylation of nucleosides, the protective groups are needed.

Biocatalysis driven by enzymes provides a higher level of stereo and regio specificity compared to chemical synthesis. The enzymes often catalyse formation of anomerically pure compounds. The enzymatic catalysis is often regioselective; therefore, the protection and deprotection of the specific groups are not needed.

According to the invention, this and other objects achieved providing a method and enzymes for the production of fucosylated nucleoside analogues. Enzymes comprise nucleic acid sequences encoding enzymes involved in fucosylated oligosaccharide hydrolysis, more specifically, enzyme gene sequences comprise a nucleic acid sequence coding fucosyl hydrolases. The conditions of the biocatalytic process are provided to achieve the previously mentioned objective, which is to obtain fucosylated nucleoside derivatives. The invention describes the method for regioselective formation of anomerically pure fucosylated nucleosides without protection and deprotection of the specific groups.

### SUMMARY OF THE INVENTION

Nucleoside analogues, including disaccharide nucleosides, are attractive biologically active compounds that can be used as antivirals or in cancer therapy. In order to use these compounds in therapeutic applications, it is essential that these compounds are both anomerically and regio-pure. Their wider diversity and availability are limited by the lack of efficient synthesis routes. Chemical methods of nucleoside synthesis require a large number of reactions, blocking-deblocking steps and sometimes the use of toxic compounds in the synthesis process. The present invention provides a comparatively mild one-step enzymatic synthesis method for the regioselective synthesis of disaccharide nucleosides, specifically fucosylated nucleosides.

The present invention relates to process during which polypeptides regioselectively catalyse the transfer of fucosyl group acceptor substrate compounds - nucleoside analogues of general formulas (1) and (5) - to their corresponding product compounds of general formulas (3) and (6) *in vitro.*

In one aspect, the invention relates to uniquely isolated genes encoding alpha-L-fucosidase selected from the group consisting of SEQ ID NO: 1 and 2.

In certain embodiments, the present invention relates to an alpha-L-fucosidase comprising a polypeptide having at least 70% sequence identity with SEQ ID NO: 1 or 2. SEQ ID NO: 1 and SEQ ID NO: 2 share 38 % sequence identity.

The level of sequence identity between a query and a subject sequence is preferably determined using a sequence alignment program, such as the ClustalW (Thompson, et. al. 1994).

In certain embodiments, the invention provides alpha-L-fucosidase polypeptides capable of transforming pyrimidine ribonucleosides of general formula (1) and fucosyl donor of general formula (2) to the corresponding products - fucosylated pyrimidine ribonucleosides of formula (3) and compound of general formula (4) *in vitro,* as depicted in Scheme 1 below:

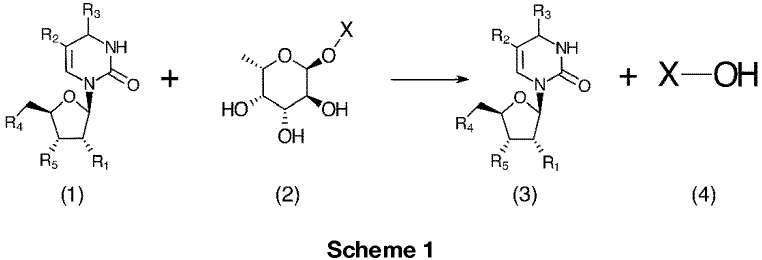

In certain embodiments, the invention provides alpha-L-fucosidase polypeptides capable of transforming purine ribonucleosides of general formula (5) and fucosyl donor of general formula (2) to the corresponding products - fucosylated purine ribonucleosides of formula (6) and compound of general formula (4) *in vitro,* as depicted in Scheme 2 below:

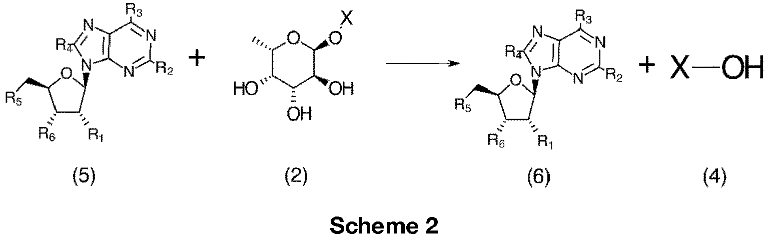

Another embodiment is a process of preparing 3'-*O*-alpha-L-fucosyl-2'-deoxyuridine comprising alpha-L-fucosidase *in vitro* wherein 2'-deoxyuridine is a fucosyl accepting substrate.

Another embodiment is a process of preparing 5'-fucosyl-2'-deoxyuridine comprising alpha-L-fucosidase *in vitro* wherein 2'-deoxyuridine is a fucosyl accepting substrate.

The details of one or more embodiments of the present invention are set forth in the following description. Other features or advantages of the present invention will be apparent from the following figures and detailed descriptions of several embodiments and the scope of the attached patent application.

### BRIEF DESCRIPTION OF FIGURES

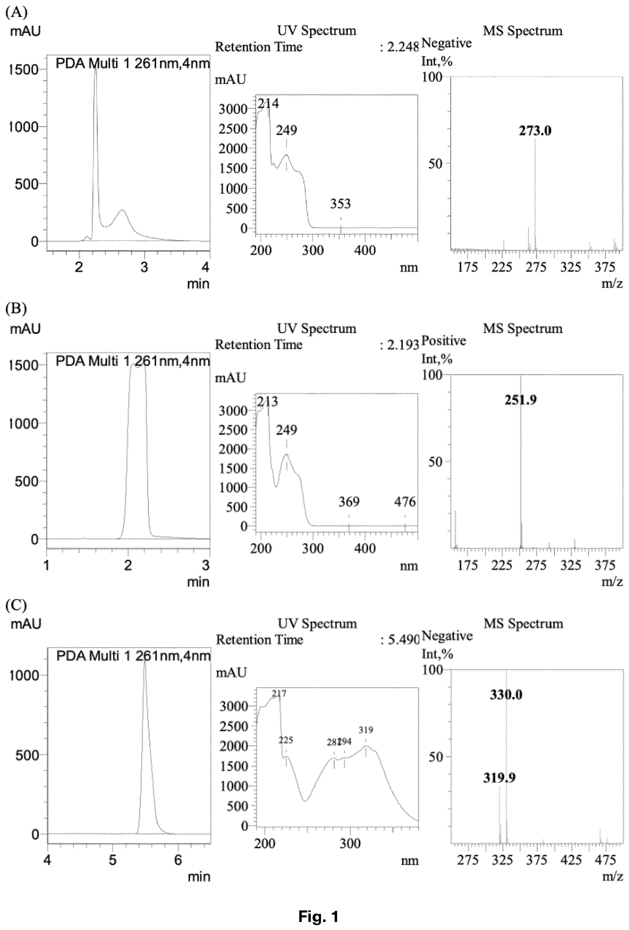

**Figure 1.** The high-performance liquid chromatography and mass spectrometry (HPLC-MS) analysis of fucosyl acceptor substrates 2'-deoxyuridine (A), 2'-deoxyadenosine (B), and fucosyl donor substrate *p*-nitrophenylfucopyranoside (C) which were used in transfucosylation reactions. UV chromatograms at 261 nm, UV-Vis absorption spectra, and MS spectra of the chemical compounds standards are presented. (A) [M + Formic acid-H⁻]⁻=273.0 Da shows 2-deoxyuridine, (B) [M + H⁺]⁺ =251.9 Da - 2-deoxyadenosine, (C) [M + Formic acid - H⁻]⁻=330.0 Da - *p-*nitrophenylfucopyranoside.

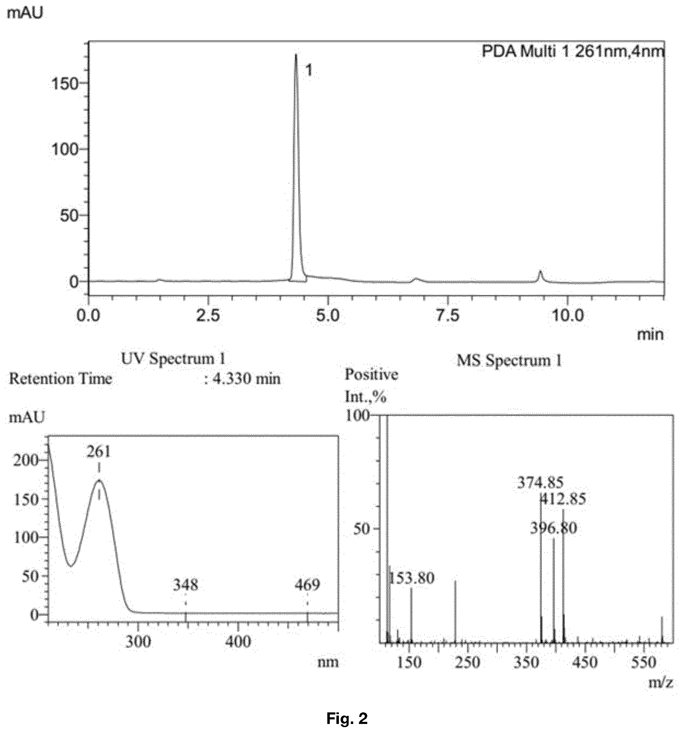

**Figure 2.** HPLC-MS analysis of the synthesized and purified 3'-*O*-alpha-L-fucosyl-2'-deoxyuridine. UV chromatogram at 261 nm, UV-Vis absorption spectrum, and MS spectra of 3'-*O*-alpha-L-fucosyl-2'-deoxyuridine are presented. 3'-*O*-alpha-L-fucosyl-2'-deoxyuridine: [M + H⁺]⁺=374.85 Da -, [M + K⁺]⁺=412.85 Da.

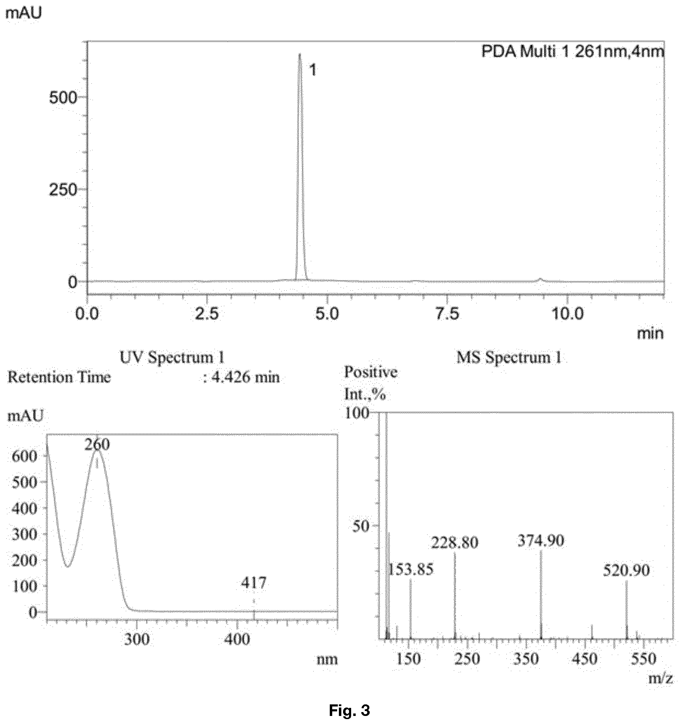

**Figure 3.** HPLC-MS analysis of the synthesized and purified 5'-*O*-alpha-L-fucosyl-2'-deoxyuridine. UV chromatogram at 261 nm, UV-Vis absorption spectrum, and MS spectra of 5'-*O*-alpha-L-fucosyl-2'-deoxyuridine are presented. 5'-*O*-alpha-L-fucosyl-2'-deoxyuridine: [M + H⁺]⁺=374.85 Da.

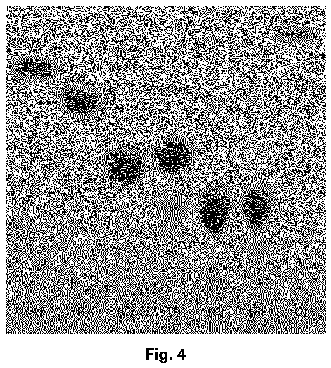

**Figure 4.** TLC analysis of fucosyl acceptor substrates: 2'-deoxyuridine (A) and 2'-deoxyadenosine (B); regioselectively synthesized and purified fucosylated nucleoside analogues: 3'-*O*-alpha-L-fucosyl-2'-deoxyuridine (C), 5'-*O*-alpha-L-fucosyl-2'-deoxyuridine (D), 3'-*O*-alpha-L-fucosyl-2'-deoxyadenosine (E), 5'-*O*-alpha-L-fucosyl-2'-deoxyadenosine (F); and fucosyl donor substrate: p-nitrophenylfucopyranoside (G).

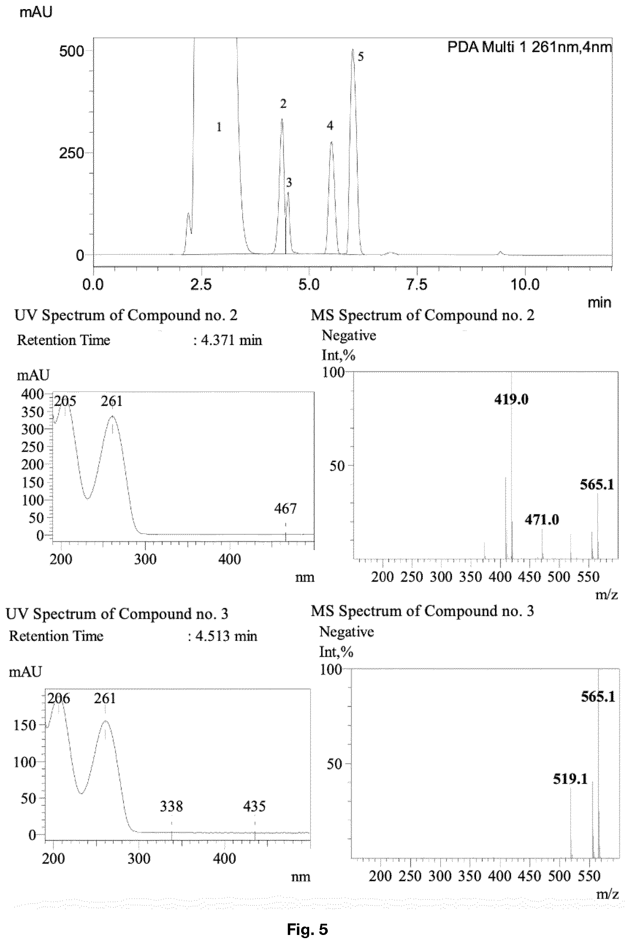

**Figure 5.** HPLC-MS analysis of reaction mixture after transfucosylation of 2'-deoxyuridine by the polypeptide of SEQ ID NO:1. The peaks in the chromatogram correspond to the following compounds: 1 - 2'-deoxyuridine, 2 - monofucosyl 2'-deoxyuridine, 3 - difucosyl 2'-deoxyuridine, 4 - *p*-nitrophenylfucopyranoside, 5 - *p*-nitrophenol. Monofucosyl 2'-deoxyuridine: [M + Formic acid-H⁻]⁻= 419.0 Da; difucosyl 2'-deoxyuridine: [M + Formic acid⁻]⁻= 565.1 Da.

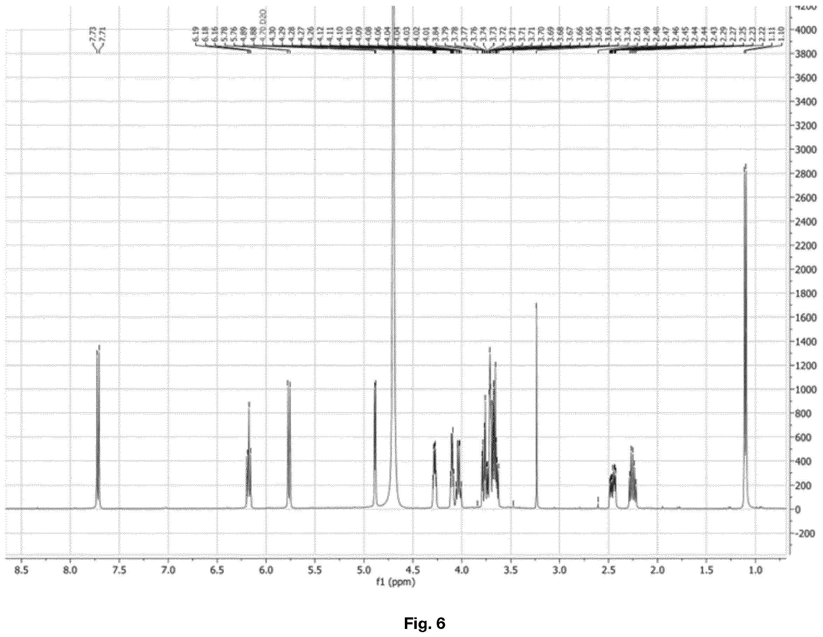

**Figure 6.** ¹H NMR profile of 3'-*O*-alpha-L-fucosyl-2'-deoxyuridine. The compound was prepared after transfucosylation of 2'-deoxyuridine by the polypeptide of SEQ ID NO:1. The compound was purified by a two-step liquid chromatography method presented in the Example No 5.

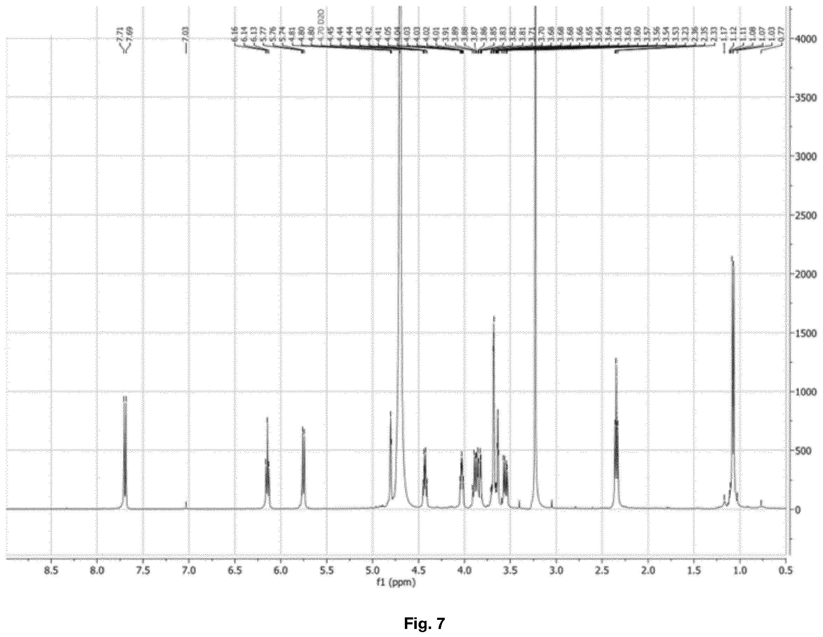

**Figure 7.** ¹H NMR profile of 5'-*O*-alpha-L-fucosyl-2'-deoxyuridine. The compound was prepared after transfucosylation of 2'-deoxyuridine by the polypeptide of SEQ ID NO:2. The compound was purified by a two-step liquid chromatography method presented in the Example No 5.

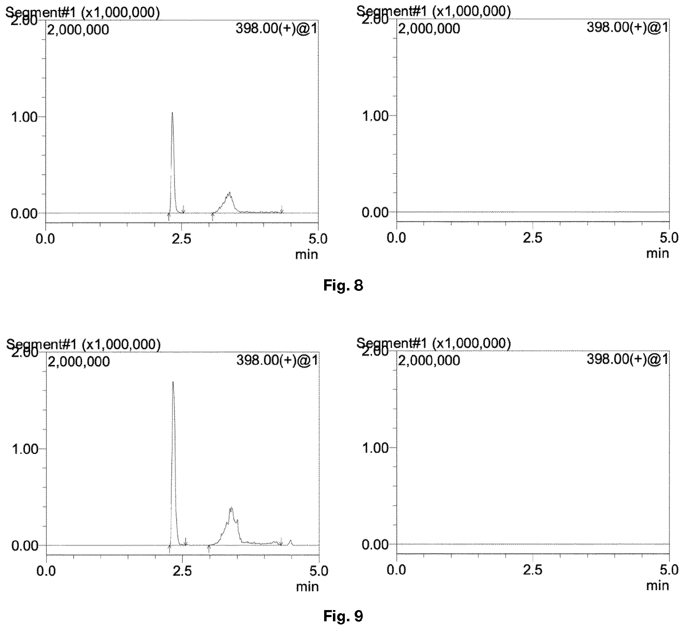

**Figure 8.** Extracted ion chromatogram of synthesized monofucosylated 2'-deoxyadenosine. HPLC-MS analyses of the reaction mixture after transfucosylation of 2'-deoxyadenosine by the polypeptide of SEQ ID NO:1 (A) and of the 2'-deoxyadenosine and *p*-nitrophenylfucopyranoside mixture incubated without polypeptide of SEQ ID NO:1 (B) were performed. Molecular ion [M+H⁺]⁺=398 Da corresponds to a monofucosylated 2'-deoxyadenosine.
**Figure 9.** Extracted ion chromatogram of synthesized monofucosylated 2'-deoxyadenosine. HPLC-MS analyses of the reaction mixture after transfucosylation of 2'-deoxyadenosine by the polypeptide of SEQ ID NO:2 (A) and of the 2'-deoxyadenosine and *p*-nitrophenylfucopyranoside mixture incubated without polypeptide of SEQ ID NO:2 (B) were performed. Molecular ion [M+H⁺]⁺=398 Da corresponds to a monofucosylated 2'-deoxyadenosine.

While the disclosure is susceptible to various modifications and alternative forms, specific embodiments thereof are shown by way of example in the drawing and will herein be described in detail. It should be understood, however, that the drawings and detailed description presented herein are not intended to limit the disclosure to the embodiment disclosed, but on the contrary, the intention is to cover all modifications, equivalents, and alternatives falling within the spirit and scope of the present disclosure as defined by the appended claims.

### DETAILED DESCRIPTION OF THE INVENTION

The term "alpha-L-fucosidases" as used therein, unless otherwise specified, refers to a polypeptide that catalyzes the transfer of fucosyl group from fucosyl donor substrate to fucosyl acceptor substrate and includes at least one of declared herein specified polypeptide of SEQ ID NO: 1 or 2.

The term "fucosyl" preferably means an L-fucopyranosyl group attached to the parent molecular moiety through a glycosidic linkage:

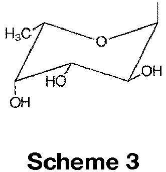

The term "donor" is to be understood as a compound that provides or transfers a specific moiety in a chemical reaction, e.g. a nucleophilic or electrophilic substitution reaction, to a further compound, preferably an acceptor. Particularly preferably, a "donor" is understood as a compound that provides or transfers a fucosyl group to a further compound, preferably an acceptor, wherein the donor is not restricted to naturally occurring donors. Likewise, the term "acceptor" is to be understood as a compound that receives a specific moiety, preferably a fucosyl moiety, in a chemical reaction, e.g. nucleophilic or electrophilic substitution reaction, from a further compound, preferably a donor as defined above.

The term "fucosyl donor substrate" is to be understood as a compound that provides fucosyl moiety and is characterized by general formula (2) in Schemes 1 and 2, wherein X, independently, is selected from the group consisting of 4-nitrophenoxy, 2-nitrophenoxy, 2,4-dinitrophenoxy, 2-chloro-4-nitrophenoxy, or lactose moiety.

The term "fucosyl acceptor substrate" is to be understood as a compound that receives fucosyl moiety and is characterized by general formula (1) or (5).

As used herein, the term "nucleoside" means a ribonucleoside or deoxyribonucleoside, or a modified form thereof, or an analogue thereof. The terms "derivative" or "compound" and other word having the same root and used in combination with the term "uridine" mean that the nucleosides have additional chemical groups that are not present in natural uridine nucleoside.

The term "transfucosylation" means any reaction that transfers a fucosyl from fucosyl donor substrate to fucosyl acceptor substrate.

The term *"in vitro"* is any system out of the cell environment that provides the conditions for production of fucosylated nucleoside analogues. It also includes aqueous systems with pH maintaining capacity and their formulations.

The term "cellular system" is any cell that is used for the expression of recombinant proteins. It includes both prokaryotic and eukaryotic cells. It also includes the *in vitro* expression of proteins based on cellular components, such as ribosomes.

The term "growing the cellular system" includes providing a medium that would allow cells to multiply and divide. It also includes providing resources so that cells or cellular components can translate and produce recombinant proteins.

Transformation is the process of deliberately introducing exogenous nucleic acids into cells. The term is often used as generic term and more often is used to describe non-viral DNA transfer into bacteria, non-animal eucaryotic cells. Transformation, more specifically electroporation, is used for this application.

### Example no. 1. Identifying alpha-L-fucosidases and cloning them into E. coli expression vector

Water samples were collected at two sites in the Curonian Lagoon (Lithuania). Cells were harvested, DNA extracted and a shotgun metagenomic analysis was carried out. Nucleotide sequences of genes in plasmids were determined at Macrogen Europe (the Netherlands) using the following sequencing primers: T7 Promoter (5'- TAATACGACTCACTATAGGG-3') and LIC Reverse Sequencing primer, 24-mer (5'-GAGCGGATAACAATTTCACACAGG-3'). Two genes encoding proteins of SEQ ID NO: 1 and 2 were identified. Using SMART (Simple Modular Architecture Research Tool) (http://smart.embl-heidelberg.de/) SEQ ID NO: 1 and 2 protein sequences were analyzed and were found to contain α-L-fucosidase domains (pfam01120). The genes encoding proteins of SEQ ID NO: 1 and 2 were amplified by PCR with specific primers (agaaggagatataactatgGACAAGATGTGGGGGGACTCGAA and gtggtggtgatggtgatggccGCGAATGAGTGTCATGCCTATG for the gene encoding SEQ ID NO: 1; agaaggagatataactatgCGAGTTCTATTGATTGCCTTTTTATTG and agaaggagatataactatgCAGAAAGATTTTTTAAATGAAACAAC for the gene encoding SEQ ID NO: 2) from pooled DNA samples using Phusion DNA polymerase (Thermo Scientific, Lithuania). The obtained DNA fragments were ligated into pLATE31 (Thermo Fisher Scientific) expression vector using aLICator LIC Cloning and Expression Kit 3 protocol (Thermo Fisher Scientific). The following reaction mixture was prepared at room temperature: 5X LIC Buffer 2 µL, purified PCR product 0.1 pmol, water, nuclease-free to 9 µL and T4 DNA Polymerase (1u/µL) 1 µL. Total mixture volume was 10 µL. The reaction mixture was incubated at room temperature (20-25°C) for 5 min. After incubation, the reaction was stopped by adding 0.6 µL of 0.5 M EDTA. Then 1 µL of pLATE31, LIC-ready vector (60 ng, 0.02 pmol DNA) was added. The mixture was incubated at room temperature (20-25°C) for 30 min. Transformation of competent DH5α *E.coli* cells (Novagen, Germany) was performed by electroporation. Finally, the cells were plated on LB agar supplemented with ampicillin. Plasmid DNA was extracted using "ZR Plasmid Miniprep - Classic" (Zymo Research) and sequenced.

### Example no. 2. Overexpression of alpha-L-fucosidases in E. coli cells and purification of alpha-L-fucosidases

Plasmids containing the genes encoding proteins of SEQ ID NO: 1 and 2 were used to transform *E*. *coli* BL21(DE3) (Novagen, Germany). *E*. *coli* BL21(DE3) bacteria cells harbouring recombinant plasmids were cultured in LB medium containing 100 µg/mL of ampicillin with shaking (180 rpm) at 30 °C until culture optical density (A600) reached 0.4-0.6. The expression was induced with 0.1 mM isopropyl β-d-1-thiogalactopyranoside (IPTG; Thermo Scientific, Lithuania), and cells were grown at 20 °C with shaking (180 rpm) overnight (20 hours). The cells were harvested by centrifugation for 10 min at 4000 *g* (4 °C), resuspended in buffer solution (50 mM potassium phosphate, 500 mM NaCl, 10 % glycerol, pH 7.5) and disrupted by sonication (2 min (5 s disruption, 10 s cooling) at 22 kHz and 40 % amplitude). The insoluble debris was removed by centrifugation (10 min, 20000 *g* 4 °C), and the clear supernatant was used for protein purification.

For the protein of SEQ ID NO: 1: Ni²⁺ HiTrap chelating HP column (Cytiva, Sweden) was equilibrated and washed with buffer A (50 mM potassium phosphate buffer, pH 7.5, containing 500 mM sodium chloride). The cell-free extract of protein of SEQ ID NO: 1 was applied onto the prepared column and, after a five-column wash with buffer solution A, the His-tagged proteins were eluted using buffer A supplemented with 500 mM imidazole. A linear elution gradient was used: length of 9 column volumes, 0-0.5 M of imidazole. Eluted fractions were pooled, and imidazole was removed using a HiTrap Desalting column (Cytiva, USA). After desalting, the proteins were concentrated by centrifugation using 30 kDa Spin-X UF filter columns (Corning, USA). The SDS-PAGE gel (5 % concentrating and 14 % resolving) was used to analyse the purified samples. The gel was stained with Coomassie Brilliant Blue R-250 and washed with a 7 % acetic acid solution. The concentrations of the purified protein solutions were determined using the Folin-Ciocalteau reagent as described by Lowry et al. (1951). Finally, the purified and concentrated proteins were supplemented with glycerol to the final concentration of 50% and stored at -20 °C.

For the protein of SEQ ID NO: 2: Ni²⁺ HiTrap chelating HP column (Cytiva, Sweden) was equilibrated and washed with buffer A (50 mM potassium phosphate buffer, pH 7.5, containing 300 mM potassium chloride). The cell-free extract of protein of SEQ ID NO: 2 was applied onto the prepared column and, after a five-column wash with buffer solution A, the His-tagged proteins were eluted using buffer A supplemented with 500 mM imidazole. A linear elution gradient was used: length of 20 column volumes, 0-0.5 M of imidazole. Eluted fractions were pooled, and imidazole was removed using a HiTrap Desalting column (Cytiva, USA). After desalting, the protein was purified using the ion exchange column (HiTrap Q XL, Cytiva, Sweden) pre-equilibrated with 5 mM Tris-HCl buffer, pH 8.0. Proteins were subsequently eluted with 15 column volumes of a linear salt gradient (0-0.5 M NaCl) in 5 mM Tris-HCl buffer, pH 8.0, at a flow rate of 0.5 ml/min. The fractions showing activity of alpha-L-fucosidase were pooled and dialyzed against 25 mM Tris-HCl buffer solution, pH 8.0. Finally, the purified protein was concentrated with carboxymethyl cellulose, supplemented with glycerol to the final concentration of 50% and stored at -20 °C. The concentrations of the purified protein solutions were determined using the Folin-Ciocalteau reagent as described by Lowry et al. (1951).

The activity of alpha-L-fucosidases was assayed using p-nitrophenyl-alpha-L-fucopyranoside as a substrate and measuring the amount of p-nitrophenol formed at 410 nm spectrophotometrically. The reaction mixture (1 ml) contained 50 mM potassium phosphate buffer (pH 7.5), p-nitrophenyl-alpha-L-fucopyranoside at concentration of 0.25 mM, and 10 µL of alpha-L-fucosidase. All reactions were performed at room temperature (20 °C) for 2 min.

### Example no. 3. Regioselective synthesis of 3'- O-alpha-L-fucosyl-2'-deoxyuridine

Regioselective synthesis of 3'-O-alpha-L-fucosyl-2'-deoxyuridine was performed using polypeptide of SEQ ID NO: 1. The working concentration of p-nitrophenyl-alpha-L-fucopyranoside (fucosyl donor substrate) was 20 mM (dissolved in DMSO) and working concentration of 2'-deoxyuridine (fucosyl acceptor substrate; dissolved in reaction buffer solution) was 60 mM. The working concentration of polypeptide of SEQ ID NO: 1 was 0.018 mg/ml. Reaction was performed in a 50 mM potassium phosphate buffer solution, pH 7.5, at 37 °C temperature under constant mixing of 180 rpm for 4-8 hours. DMSO concentration did not exceed 5 % in the final reaction mixture. Reaction was performed in a 50 mL glass flask and the final volume of reaction mixture did not exceed 20 ml.

### Example no. 4. Regioselective synthesis of 5'- O-alpha-L-fucosyl-2'-deoxyuridine

Regioselective synthesis of 5'-*O*-alpha-L-fucosyl-2'-deoxyuridine was performed using polypeptide of SEQ ID NO: 2. The working concentration of *p*-nitrophenyl-alpha-L-fucopyranoside (fucosyl donor substrate) was 20 mM (dissolved in DMSO) and working concentration of 2'-deoxyuridine (fucosyl acceptor substrate; dissolved in reaction buffer solution) was 60 mM. The working concentration of polypeptide of SEQ ID NO: 2 was 0.06 mg/ml. Reaction was performed in a 50 mM potassium phosphate buffer solution, pH 7.5, at 37 °C temperature under constant mixing of 180 rpm for 22 hours. DMSO concentration did not exceed 5 % in the final reaction mixture. Reaction was performed in a 50 mL glass flask and the final volume of reaction mixture did not exceed 20 ml.

### Example no. 5. Purification of fucosylated 2'-deoxyuridine

Purification process consisted of 2 steps: the 1st step was for partial removal of the unreacted fucosyl acceptor substrate and p-nitrophenyl-alpha-L-fucopyranoside, the 2nd step was for final removal of the unreacted fucosyl acceptor substrate and p-nitrophenyl-alpha-L-fucopyranoside and to isolate 3'-*O*-alpha-L-fucosyl-2'-deoxyuridine and 5'-*O*-alpha-L-fucosyl-2'-deoxyuridine. Step 1: The samples were concentrated 2-fold using vacuum rotary evaporator (from 20 ml to 10 ml). Mobile phase A used for purification was water. Mobile phase B used for purification was methanol. Concentrated samples were loaded onto the Biotage Sfar C18 D Duo 100 Å column. Column was washed with 5 CV of mobile phase A. Eight CV linear gradient (0-100 %) of mobile phase B was applied for compound elution. The collected fractions were analyzed by TLC and HPLC-MS. The fractions with two different fucosylated products were pooled separately. The pooled fractions were concentrated using vacuum rotary evaporator to dryness and were diluted to 2.0 mL with water.

Step 2: The fractions from step 1 with partially purified 3'-*O*-alpha-L-fucosyl-2'-deoxyuridine and 5'-*O*-alpha-L-fucosyl-2'-deoxyuridine were loaded onto the Biotage Sfar C18 D Duo 100 Å column separately. The column was washed with 2 CV of mobile phase A and compounds were eluted with mobile phase B linear gradient (0-100 %) of 3 CV. The fractions were evaluated using HPLC-MS and concentrated using vacuum rotary evaporator. The NMR analyses were performed.

### Example no. 6. Regioselective synthesis of 3'- O-alpha-L-fucosyl-2'-deoxyadenosine

Regioselective synthesis of 3'-*O*-alpha-L-fucosyl-2'-deoxyadenosine was performed using polypeptide of SEQ ID NO: 1. The working concentration of *p*-nitrophenyl-alpha-L-fucopyranoside (fucosyl donor substrate) was 20 mM (dissolved in DMSO) and working concentration of 2'-deoxyadenosine (fucosyl acceptor substrate; dissolved in DMSO) was 60 mM. The working concentration of polypeptide of SEQ ID NO: 1 was 0.018 mg/ml. Reaction was performed in a 50 mM potassium phosphate buffer solution, pH 7.5, at 37 °C temperature under constant mixing of 180 rpm for 4-8 hours. DMSO concentration did not exceed 10 % in the final reaction mixture. Reaction was performed in a 50 mL glass flask and the final volume of reaction mixture did not exceed 20 ml.

### Example no. 7. Regioselective synthesis of 5'- O-alpha-L-fucosyl-2'-deoxyadenosine

Regioselective synthesis of 5'-*O*-alpha-L-fucosyl-2'-deoxyadenosine was performed using polypeptide of SEQ ID NO: 2. The working concentration of p-nitrophenyl-alpha-L-fucopyranoside (fucosyl donor substrate) was 20 mM (dissolved in DMSO) and working concentration of 2'-deoxyadenosine (fucosyl acceptor substrate; dissolved in DMSO) was 60 mM. The working concentration of polypeptide of SEQ ID NO: 2 was 0.06 mg/ml. Reaction was performed in a 50 mM potassium phosphate buffer solution, pH 7.5, at 37 °C temperature under constant mixing of 180 rpm for 22 hours. DMSO concentration did not exceed 10 % in the final reaction mixture. Reaction was performed in a 50 mL glass flask and the final volume of reaction mixture did not exceed 20 ml.

### Example no. 8. Purification of fucosylated 2'-deoxyadenosine

Mobile phase A used for purification was water. Mobile phase B used for purification was methanol. The samples after transfucosylation of 2'-deoxyadenosine by polypeptide of SEQ ID NO:1 OR 2 were loaded onto the Biotage Sfar C18 D Duo 100 Å column. Column was washed with 13 CV of mobile phase A, then with 15 CV of 10 % mobile phase B. Ten CV linear gradient (10-50 %) of mobile phase B followed with 10 CV of 100 % mobile phase B was applied. The collected fractions were analyzed by TLC and HPLC-MS. The fractions with two different fucosylated products were pooled separately. The pooled fractions were concentrated using vacuum rotary evaporator to dryness.

### Example no. 9. Analysis of fucosylated nucleosides

### Assay of fucosylated product detection by a thin-layer chromatography method

The progress of the transfucosylation reaction and the degree of fucosylated nucleoside purification were evaluated by thin layer chromatography. Silica gel 60 chromatography plates with fluorescent indicator F254 (Merck, USA) were used for compound separation. The aliquots of reaction mixtures or purification fractions were added on the thin layer chromatography plates as spots. The mobile phase consisted of ethyl acetate, methanol, and H₂O at a ratio of 7:2:1, respectively. After the separation, the plates were dried. Compounds were visualized using UV light exposure of 254 nm.

### HPLC-MS analysis of transfucosylation reaction mixtures

The progress of the transfucosylation reaction was analysed using a high-pressure liquid chromatography system (Shimadzu, Japan) equipped with a photodiode array (PDA) detector (Shimadzu, Japan) and mass spectrometer (LCMS-2020; Shimadzu, Japan) with an electrospray ionization (ESI) source. The chromatographic separation was conducted using a YMC Pack Pro column (3 × 150 mm; YMC, Japan) at 40°C and a mobile phase that consisted of 0.1 % formic acid water solution (solvent A) and acetonitrile (solvent B) delivered in the 5-95% gradient elution mode. Mass scans were measured from m/z 50 up to m/z 1,200 at a 350°C interface temperature, 250°C desolvation line (DL) temperature, ±4,500 V interface voltage, and neutral DL/Qarray, using N₂ as nebulizing and drying gas. Mass spectrometry data were acquired in both positive and negative ionization modes. The data were analyzed using LabSolutions liquid chromatography-mass spectrometry (LCMS) software. Prior the analyses, the samples were mixed with acetonitrile in a ratio 1:1 and centrifuged at 10,000 × *g* for 5 min.

### NMR analysis of fucosylated 2'-deoxiuridines

¹H NMR, ¹³C NMR, COSY, HMQC and HMBC spectra were recorded in D₂O, with the addition of a drop of methanol, on Avance III 400 NMR spectrometer, at 400 MHz for ¹H and 100 MHz for ¹³C. Chemical shifts are reported in ppm relative to methanol resonance signal as an internal standard ( ¹H NMR: δ (CH₃OH) = 3.34ppm; ¹³C NMR: δ (CH₃OH) = 49.50 ppm).

The molecule synthesized by polypeptide of SEQ ID NO: 1 was identified as 3'-*O*-α-L-fucosyl-2'-deoxyuridine (Scheme 4):

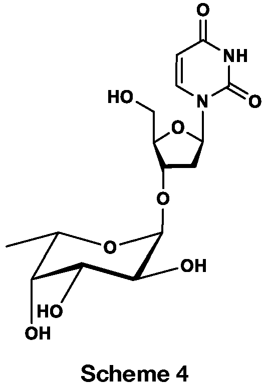

¹H NMR (400 MHz, D₂O) δ 7.82 (d, *J* = 8.1 Hz, 1H, H-6), 6.28 (t, *J* = 6.8 Hz, 1H, H-1 '), 5.87 (d, *J* = 8.0 Hz, 1H, H-5), 4.99 (d, *J* = 4.0 Hz, 1H, H-1"), 4.38 (dt, *J* = 6.8, 3.6 Hz, 1H, H-3'), 4.20 (dd, *J* = 8.3, 3.9 Hz, 1H, H-4'), 4.14 (q, *J* = 6.6 Hz, 1H, H-5"), 3.90 - 3.73 (m, 5H, H-2", H-3", H-4", H-5a', H-5b'), 2.56 (ddd, *J* = 14.2, 6.3, 3.6 Hz, 1H, H-2a'), 2.36 (dt, *J* = 14.0, 6.8 Hz, 1H, H-2b'), 1.21 (d, *J* = 6.6 Hz, 3H, H-6").

¹³C NMR (100 MHz, D₂O) δ 168.0 (C-4), 153.0 (C-2), 142.4 (C-6), 102.9 (C-5), 99.3 (C-1"), 86.4 (C-1'), 85.4 (C-4'), 78.3 (C-3'), 72.4 (C-4"), 70.0 (C-3"), 68.5 (C-2"), 67.9 (C-5"), 62.0 (C-5'), 37.8 (C-2'), 15.9 (C-6").

The molecule synthesized by polypeptide of SEQ ID NO: 2 was identified as 5'-*O*-α-L-fucosyl-2'-deoxyuridine (Scheme 5):

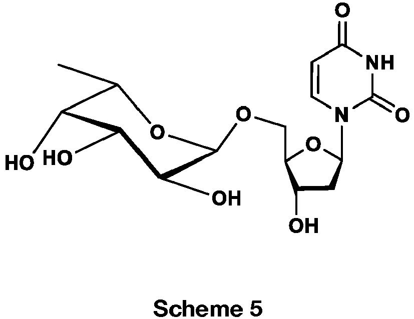

¹H NMR (400 MHz, D₂O) δ 7.81 (d, J = 8.1 Hz, 1H, H-6), 6.26 (t, J = 6.4 Hz, 1H, H-1'), 5.86 (d, J = 8.0 Hz, 1H, H-5), 4.97 - 4.87 (m, 1H, H-1"), 4.54 (dd, J = 10.7, 5.8 Hz, 1H, H-3'), 4.22 - 4.07 (m, 1H, H-4'), 4.07 - 3.88 (m, 2H, H-5",H-5a'), 3.83 - 3.77 (m, 2H, H-2", H-3"), 3.77 - 3.73 (m, 1H, H-4"), 3.66 (dd, J = 11.4, 5.3 Hz, 1H, H-5b'), 2.46 (t, J = 6.2 Hz, 2H, H-2a',H-2b'), 1.19 (d, J = 6.6 Hz, 3H, H-6").

¹³C NMR (100 MHz, D₂O) δ 168.0 (C-4), 153.0 (C-2), 142.5 (C-6), 102.7 (C-5), 99.6 (C-1"), 86.3 (C-1'), 85.6 (C-4'), 72.4 (C-4"), 70.8 (C-3'), 70.2 (C-3"), 68.6 (C-2"), 67.8 (C-5'), 67.4 (C-5"), 38.9 (C-2'), 15.9 (C-6").

As is evident from the foregoing description, certain aspects of the present disclosure are not limited by the particular details of the examples illustrated herein, and it is therefore contemplated that other modifications and applications, or equivalents thereof, will occur to those skilled in the art. It is accordingly intended that the claims shall cover all such modifications and applications that do not depart from the spirit and scope of the present disclosure.

Moreover, unless defined otherwise, all technical and scientific terms used herein have the same meaning as commonly understood by one of ordinary skill in the art to which the disclosure belongs. Although any methods and materials similar to or equivalent to or those described herein can be used in the practice or testing of the present disclosure, the preferred methods and materials are described above.

Other aspects, objects, and advantages of the present disclosure can be obtained from a study of the drawings, the disclosure and the appended claims.

### List of sequences

| | |
|---|---|
| SEQ ID No: I | MDKMWGDSKSKATQSDLTRGPSLREGRYGMFIHWGLYSSLGGKWKDKTYYGIGEWI MNPAMAGIPVEDYMEPAKSFNPEQFDAKAIVQLAKDAGMKYIIITSKHHEGFAMFKSA HPFNIVDASPFQRDPMKELSDACRAAGLGFGFYYSHFQDWTSPGGHGGPSKNADGSD ATFEKYFREKCYPQVKEICTQYGPLDVIWFDTPGSMPKELVVELHDLVRSTQPKALLG SRIGHGMGDYESLGDMEVPPERVDGFWESCDTTNDSWSYAWYDTNWKSPKVILHRL VSTVARGGNYLLNIGPDGKGAVPAECQEFLKQAGTWLKAHPEVISESGPSPWRYAMP WGDITVRKDGKLNLVVFDLPQDGFIYLPGLSTPVKSASLIDTGRKTLVKTELFGKSMRI KLPERCRTAIATLVELQLTSAPEVDQSVTVHPNTPNDLLVHFAEVMGAEKKKTNWMA KFGEWKHATQISRWTPGGKARWTANFLQPGFYQVTAKYRGEMDPKSRVTWRITTNE ANSIQNQQPVTGQYQSMPMGVFEVKMPGKRVIEVSLVDGDLDATSLESIGMTLIR |
| SEQ ID No:2 | MQKDFLNETTQDRDARMQWWRDATFGMFIHWGAYAVPAGEYKGKVVNGVGEWIM HTANIPIPEYEEYVRQFNPLQFDAKEWVRIAKDAGMKYIVITSKHHDGFSLWDSKVSN YDVMDASPFKRDILKELSDACKEAGIKMCFYHSIMDWHEPDAESKKEYTHQNAPNPD FARYREQFLKPQLKELVEKYDPAVLWFDGEWIPEWTEEQGRDLYNYLRNLKPDLVIN NRVGKGRAGMQGMNLYKNAAGDFGTPEQEILEGTSDTDWESCMTMNDSWGFKKTD HNWKSAELLIHNLVDIAAKGGNYLLNVGPTAQGLIPVASVDRLREMGEWMKINNEAI YATNSTKKYKEGEVKYTVSKDGNFVYAIVTKAVSGELVLHNVDLKRGSNIYMLGAN KPLKYKADASGSLHIEWPKDLPCKYAWTLKITGSPK |
| T7Pro Primer | TAATACGACTCACTATAGGG |
| LICRev Primer | GAGCGGATAACAATTTCACACAGG |
| fucIF Primer | AGAAGGAGATATAACTATGGACAAGATGTGGGGGGACTCGAA |
| fuc1R Primer | GTGGTGGTGATGGTGATGGCCGCGAATGAGTGTCATGCCTATG |
| fuc2F Primer | AGAAGGAGATATAACTATGCGAGTTCTATTGATTGCCTTTTTATTG |
| fuc2R Primer | AGAAGGAGATATAACTATGCAGAAAGATTTTTTAAATGAAACAAC |

### References

1. Guinan M, Benckendorfif C, Smith M, Miller GJ. Recent Advances in the Chemical Synthesis and Evaluation of Anticancer Nucleoside Analogues. Molecules, 2020; 25(9):2050. https://doi.org/10.3390/molecules25092050.
2. Yates MK, Seley-Radtke KL. The evolution of antiviral nucleoside analogues: A review for chemists and non-chemists. Part II: Complex modifications to the nucleoside scaffold. Antiviral Research, 2019, 162: 5-21, https://doi.org/10.1016/j.antiviral.2018.11.016.
3. Liu Y, Jia Y, Yang K. et al. Anti-HIV agent azidothymidine decreases Tet(X)-mediated bacterial resistance to tigecycline in Escherichia coli. Commun Biol, 2020; 3, 162. https://doi.org/10.1038/s42003-020-0877-5.
4. Sibley D, Larkin D.F.P. Update on Herpes simplex keratitis management. Eye, 2020; 34, 2219-2226. https://doi.org/10.1038/s41433-020-01153-x
5. loka T, Shindo Y, Ueno M, Nagano H. Current progress in perioperative chemotherapy for biliary tract cancer. Ann Gastroenterol Surg, 2023; 7: 565-571. https://doi.org/10.1002/ags3.12691.
6. Siedentop R, Rosenthal K. Industrially Relevant Enzyme Cascades for Drug Synthesis and Their Ecological Assessment. International Journal of Molecular Sciences, 2022; 23(7):3605. https://doi.org/10.3390/ijms23073605.
7. Someya H, Itoh T, Aoki S. Synthesis of Disaccharide Nucleosides Utilizing the Temporary Protection of the 2',3'-cis-Diol of Ribonucleosides by a Boronic Ester. Molecules, 2017; 22(10):1650. https://doi.org/10.3390/molecules22101650
8. Thompson JD, Higgins DG, Gibson TJ. CLUSTAL W: improving the sensitivity of progressive multiple sequence alignment through sequence weighting, position-specific gap penalties and weight matrix choice. Nucleic Acids Research, 1994; 22 (22), 4673-4680. https://doi.org/10.1093/nar/22.22.4673

## Claims

1. A method for synthesis of fucosylated nucleosides with alpha-L-fucosidases, comprising:
a) Selecting the nucleic acid from the group encoding SEQ ID NO: 1 and 2;
b) Constructing a DNA vector harboring the selected nucleic acid;
c) Transforming the host cell with the DNA vector from step (b);
d) Expressing and purifying polypeptides of SEQ ID NO: 1 and 2;
e) Transfucosylating nucleoside derivative to a fucosylated nucleoside derivative.

2. The method according to claim 1, wherein alpha-L-fucosidase is isolated.

3. The method according to claim 1, wherein isolated alpha-L-fucosidase with an amino acid sequence from the group SEQ ID NO: 1 and 2 transfucosylates a nucleoside to a fucosylated nucleoside *in vitro.*

4. The method according to claims 1-3, wherein alpha-L-fucosidase comprising a polypeptide having at least 70% sequence identity with SEQ ID NO: 1 or 2.

5. The method according to claim 1, wherein nucleoside derivative is pyrimidine ribonucleoside or purine ribonucleoside.

6. The method according to claim 1, wherein fucosylated nucleoside derivative is fucosylated pyrimidine ribonucleosides or fucosylated purine ribonucleosides.

7. The method according to claim 1-6, wherein pyrimidine ribonucleosides used for preparation of fucosylated ribonucleosides have the following general formula: 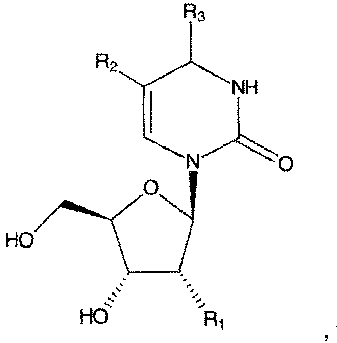 wherein
R₁ is OH or H
R₂ is Cl, Br, F, I, H, CH₃
R₃ is OH, NH₂

8. The method according to claim 1, wherein purine ribonucleosides used for preparation of fucosylated ribonucleosides have the following general formula: 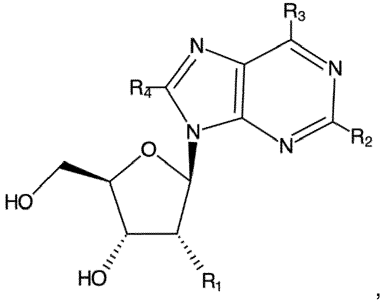 wherein
R₁ is OH or H
R₂ is Cl, F, H
R₃ is NH₂, O, Cl
R₅ is Cl, NH₂, Br

9. The method according to claim 1, wherein synthesized 5'-fucosyl-2'-deoxyuridine have the following general formula:

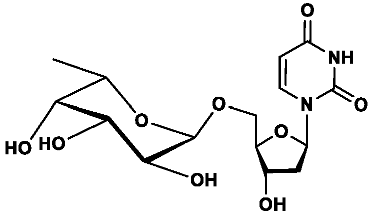

10. The method according to claim 1, wherein synthesized 3'-*O*-alpha-L-fucosyl-2'-deoxyuridine have the following general formula:
